# FASCICULE DE BREVET EUROPEEN

(11) **EP 0 721 927 B1**
(45) Date de publication et mention de la délivrance du brevet: **28.04.1999**
(21) Numéro de dépôt: 96400029.3
(22) Date de dépôt: 05.01.1996
(51) Int. Cl.: C07C 17/42, C07C 19/03, C23G 5/028

(54) **Composition à base de chlorure de Méthylène stabilisé utilisable pour le dégraissage des métaux**
Stabilisierte Methylenchlorid-Zusammensetzung und deren Verwendung zum Entfetten von Metallen
Stabilized methylene chloride composition and its use in degreasing metals

(30) Priorité: 10.01.1995 FR 9500194
(43) Date de publication de la demande: 17.07.1996
(73) Titulaire: ELF ATOCHEM S.A., 92800 Puteaux,Hauts-de-Seine (FR)
(72) Inventeur: Michaud, Pascal, F-95210 Saint-Gratien (FR)

(56) Documents cités:
- EP-A- 0 256 903
- FR-A- 2 241 520
- FR-A- 2 268 773
- FR-A- 2 601 701
- US-A- 3 646 229
- US-A- 3 670 036

## Description

La présente invention concerne une composition à base de chlorure de méthylène stabilisé utilisable pour le dégraissage, le nettoyage et/ou le séchage des pièces métalliques.

Les hydrocarbures halogénés, notamment, les hydrocarbures chlorés grâce à leur pouvoir solvant, leur ininflammabilité et leur point d'ébullition relativement bas sont très employés pour le dégraissage des métaux.

Parmi les hydrocarbures chlorés, le chlorure de méthylène s'avère être particulièrement stable à l'oxydation, à l'hydrolyse et à la pyrolyse. Son point d'ébullition bas permet son utilisation à faible température.

Toutes ces propriétés en font par conséquent un solvant de choix pour le dégraissage, le nettoyage et/ou le séchage des pièces métalliques.

Cependant, lorsque le chlorure de méthylène est utilisé dans le dégraissage, le nettoyage et/ou le séchage de certaines pièces métalliques, il peut réagir avec notamment des composés aromatiques introduits lors de l'usinage desdites pièces métalliques.

Cette réaction se manifeste tout particulièrement lorsque lesdites pièces métalliques sont en aluminium ou ses alliages et en magnésium ou ses alliages.

Sans que la demanderesse soit tenue par une quelconque explication, cette réaction se traduit par une décomposition plus ou moins importante du chlorure de méthylène avec notamment formation de composés acides tels que l'acide chlorhydrique ou le phosgène.

Cette décomposition entraîne une diminution importante de la propriété solvante du chlorure de méthylène et une modification importante du pH.

Les composés acides libérés peuvent en outre corroder les pièces métalliques et l'installation.

Cette décomposition est plus marquée encore lorsqu'on utilise le chlorure de méthylène pour des opérations de dégraissage ou de séchage à chaud.

Cette décomposition du chlorure de méthylène constitue par conséquent un grave inconvénient sur le plan de son utilisation comme solvant de dégraissage, de nettoyage et/ou de séchage de pièces métalliques constituées en métaux dits légers tels que l'aluminium ou le magnésium (ou leurs alliages).

Aussi pour pallier ces inconvénients de très nombreux composés ont été préconisés comme agents inhibiteurs de la décomposition, ou stabilisants du chlorure de méthylène.

Parmi les composés les plus utilisés, on peut citer les alcools saturés et insaturés, les cétones, les époxydes, les amines, les éthers et leurs mélanges.

Ainsi le brevet US 3 923 912 préconise d'utiliser la méthyléthylcétone.

Le brevet US 3 670 036 décrit une composition de chlorure de méthylène stabilisée par :
- 1 à 10% d'un nitroalcane ayant de 1 à 6 atomes de carbone,
- 0,1 à 5% d'un oxyde d'alkylène ayant de 2 à 4 atomes de carbone, et
- 0 à 10% d'un dialcoxyalcane ayant jusqu'à environ 20 atomes de carbone.

Bien que l'utilisation des nitroalcanes apporte une certaine action préventive contre la décomposition du chlorure de méthylène en contact avec des métaux, cette prévention est très insuffisante lorsque l'on désire protéger non seulement la phase liquide mais également la phase vapeur.

En outre, beaucoup de composés précédemment mentionnés, utilisés seuls ou en combinaison, ne permettent pas de passer le test très sévère de stabilisation longue durée dit test du BAM (Bundesanstalt für Materialprüfung).

On a maintenant trouvé une composition à base de chlorure de méthylène stabilisé, utilisable pour le dégraissage, le nettoyage à froid, comme à chaud et/ou le séchage de pièces métalliques, caractérisée en ce qu'elle est essentiellement constituée du chlorure de méthylène, par par au moins un 1,2-époxyalcane et par au moins un acétal.

A titre de 1,2-époxyalcanes utilisables selon la présente invention on citera le 1,2-époxypropane, le 1,2-époxybutane, le 1,2-époxyhexane, le 1,2-époxyoctane, le 7-oxabicyclo[4.1.0]heptane.

La présente invention concerne tout particulièrement l'utilisation du 1,2-époxybutane.

A titre d'acétals utilisables selon la présente invention on citera le diméthoxyméthane, le 1,1-diméthoxy- propane, le 1,1-diéthoxybutane, le 1,1-diméthoxyhexane, le diéthoxyméthane, le 1,3-dioxolanne.

La présente invention concerne tout particulièrement l'utilisation du diméthoxyméthane communément appelé méthylal.

Selon la présente invention, on utilise une quantité de 1,2-époxyalcane comprise entre 0,0001% et 16,50% en poids et, de préférence, une quantité comprise entre 0,2% et 2% en poids par rapport au poids total de la composition à base de chlorure de méthylène.

Selon la présente invention, on utilise une quantité d'acétal comprise entre 0,55% et 16,50% en poids et, de préférence, une quantité comprise entre 0,55% et 3% en poids par rapport au poids total de la composition à base de chlorure de méthylène.

La composition de chlorure de méthylène stabilisé peut également contenir un ou plusieurs additifs choisis parmi les amines telles que la diméthyléthylamine, la méthyldiéthylamine, la triméthylamine ; les aminoalcools tels que la triéthanolamine ; les cétones telles que l'acétone, la méthyléthylcétone ; les nitroalcanes tels que le nitrométhane, le nitropropane ; les éthers tels que le diéthyléther, le tétrahydrofuranne.

Ces additifs peuvent être utilisés en des quantités comprises entre 0,001% et 5% en poids, par rapport au poids total de la composition de chlorure de méthylène.

La composition selon l'invention peut être préparée par simple mélange des composés avec le chlorure de méthylène.

Ce mélange peut être réalisé à température ambiante et, plus spécialement, à une température comprise entre 5°C et 30°C.

La composition selon l'invention est utilisable notamment pour le dégraissage à froid, comme à chaud, le nettoyage et/ou le séchage de pièces métalliques.

La composition de l'invention présente l'avantage d'être une composition simple, possédant une très bonne stabilisation longue durée. Elle est également très stable lorsqu'elle est utilisée, notamment pour les opérations de dégraissage en phase vapeur de pièces métalliques.

] Les exemples qui suivent illustrent l'invention.

### Exempte 1 (selon l'invention)

On prépare une composition de chlorure de méthylène constituée de 98,37% en poids de CH₂CI₂, de 0,63% en poids de 1,2-époxybutane et de 1% en poids de méthylal. Cette composition est désignée ci-après par composition (I).

Cette composition est soumise au test du BAM qui comprend les essais suivants :

### - essai 1

A 25 ml de la composition (I) on ajoute :
- 25 ml de toluène,
- 0,175 g de chlorure d'aluminium et
- 4,5 g de paillettes d'aluminium.

### - essai 2

Au mélange de l'essai 1, on ajoute 2,5 g de stéarate de zinc.

### - essai 3

Au mélange de l'essai 1, on ajoute 2,5 ml d'acide oléique.

Pour chaque essai, les mélanges sont portés à 80°C au bain marie pendant 18 heures.

Ensuite on distille 300 ml de la composition (I) en 3 fractions d'environ 100 ml. La fraction 1 correspond aux têtes de distillation, la fraction 2 correspond au coeur de distillation, la fraction 3 correspond au pied de distillation.

On prélève 25 ml de chaque fraction et on applique l'essai 1 mentionné ci-dessus en respectant les quantités indiquées.

On admet que la composition (ou le solvant) satisfait au test du BAM s'il n'y a eu "aucune réaction" enregistrée au cours de chacun des six essais considérés séparément (essais 1, 2 et 3 puis essai 1 sur les fractions 1, 2 et 3). [0041 ] Par "aucune réaction", il faut comprendre aucune réaction violente exothermique, telle que décomposition ou explosion.

Les résultats obtenus sont rassemblés dans le tableau 1.

On constate que la composition (I) passe le test du BAM.

Pour vérifier l'aptitude de la composition (I) à piéger l'acide chlorhydrique, nous avons soumis la composition (1) au test dit de l' "acid acceptance" selon la norme ASTM D 2942. L' "acid acceptance" mesuré en équivalent poids de NaOH est de 0,35.

### Exemple 2 (non conforme à l'invention)

On prépare une composition de chlorure de méthylène constituée de 98,87% en poids de chlorure de méthylène, de 0,63% en poids de 1,2-époxybutane et de 0,50% en poids de méthylal. Cette composition est désignée ci-après par composition (II).

On effectue sur cette composition (II) le test du BAM.

Les résultats sont rapportés dans le tableau 1. On constate que la composition (II) ne passe pas le test du BAM.

On observe une décomposition de la fraction 2 qui a été soumise à l'essai 1.

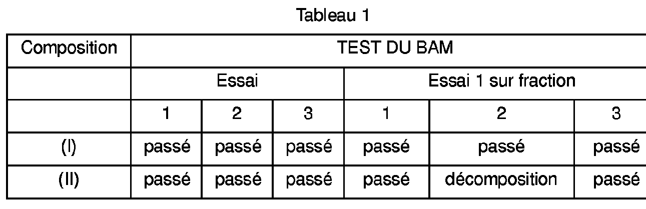

### Exemple 3

On remplit le bouilleur et la cuve de rinçage d'une petite machine de dégraissage avec des quantités égales de la composition (I).

Après une heure de reflux, lorsque le système est en équilibre, on prélève un aliquote dans le bouilleur et dans la cuve de rinçage.

] Ces deux aliquotes sont analysés par chromatographie en phase gazeuse.

Les résultats sont rassemblés dans le tableau 2.

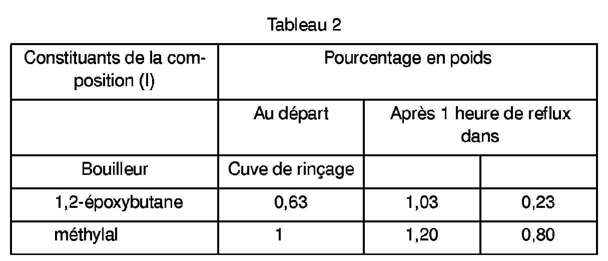

La cuve de rinçage est équivalente au retour des condensats, par conséquent, les résultats obtenus indiquent que la phase vapeur est bien stabilisée.

## Revendications

1. Utilisation pour le nettoyage et le dégraissage à chaud de pièces métalliques d'une composition à base de chlorure de méthylène, caractérisée en ce que ladite composition est constituée par du chlorure de méthylène, par au moins un 1,2-époxyalcane et par au moins un acétal.

2. Utilisation d'une composition selon la revendication 1, caractérisée en ce que le 1,2-époxyalcane est le 1,2-époxybutane.

3. Utilisation d'une composition selon la revendication 1, caractérisée en ce que l'acétal est le diméthoxyméthane.

4. Utilisation d'une composition selon l'une des revendications 1 ou 2, caractérisée en ce qu'on utilise une quantité de 1,2-époxyalcane comprise entre 0,0001 % et 16,50 % en poids par rapport au poids total de la composition à base de chlorure de méthylène.

5. Utilisation d'une composition selon la revendication 4, caractérisée en ce que la quantité de 1,2-époxyalcane est comprise entre 0,2 % et 2 % en poids.

6. Utilisation d'une composition selon l'une des revendications 1 ou 3, caractérisée en ce qu'on utilise une quantité d'acétal comprise entre 0,55 % et 16,50 % en poids par rapport au poids total de la composition à base de chlorure de méthylène.

7. Utilisation d'une composition selon la revendication 6, caractérisée en ce que la quantité d'acétal est comprise entre 0,55 % et 3 % en poids.

## Claims

1. Use for hot cleaning and degreasing of metal components of a composition based on methylene chloride, characterized in that the said composition is composed of methylene chloride, of at least one 1,2-epoxyalkane and of at least one acetal.

2. Use of a composition according to Claim 1, characterized in that the 1,2-epoxyalkane is 1,2-epoxybutane.

3. Use of a composition according to Claim 1, characterized in that the acetal is dimethoxymethane.

4. Use of a composition according to either of Claims 1 and 2, characterized in that use is made of an amount of 1,2-epoxyalkane of between 0.0001 % and 16.50 % by weight with respect to the total weight of the composition based on methylene chloride.

5. Use of a composition according to Claim 4, characterized in that the amount of 1,2-epoxyalkane is between 0.2 % and 2 % by weight.

6. Use of a composition according to either of Claims 1 and 3, characterized in that use is made of an amount of acetal of between 0.55 % and 16.50 % by weight with respect to the total weight of the composition based on methylene chloride.

7. Use of a composition according to Claim 6, characterized in that the amount of acetal is between 0.55 % and 3 % by weight.

## Patentansprüche

1. Verwendung einer Zusammensetzung auf Basis von Methylenchlorid zur Reinigung und zum Heißentfetten von Metallteilen, dadurch gekennzeichnet, daß die Zusammensetzung aus Methylenchlorid, mindestens einem 1,2 Epoxyalkan und mindestens einen, Azetal besteht.

2. Verwendung einer Zusammensetzung nach Anspruch 1, dadurch gekennzeichnet, daß das 1,2-Epoxyalkan 1,2-Epoxybutan ist.

3. Verwendung einer Zusammensetzung nach Anspruch 1, dadurch gekennzeichnet, daß das Azetal Dimethoxymethan ist.

4. Verwendung einer Zusammensetzung nach einem der Ansprüche 1 oder 2, dadurch gekennzeichnet, daß man eine Menge von 1,2-Epoxyalkan zwischen 0,0001 und 16,50 Gew.-%, bezogen auf die Gesamtmenge der Zusammensetzung auf Basis von Methylenchlorid verwendet.

5. Verwendung einer Zusammensetzung nach Anspruch 4, dadurch gekennzeichnet, daß die Menge an 1,2-Epoxyalkan zwischen 0,2 und 2 Gew.-% liegt.

6. Verwendung einer Zusammensetzung nach einen, der Ansprüche 1 oder 3, dadurch gekennzeichnet, daß man eine Menge des Azetals zwischen 0,55 und 16,50 Gew.-%, bezogen auf die Gesamtmenge der Zusammensetzung auf Basis von Methylenchlorid verwendet.

7. Verwendung einer Zusammensetzung nach Anspruch 6, dadurch gekennzeichnet, daß die Menge an Azetal zwischen 0,55 und 3 Gew.-% liegt.
